Europäisches Patentamt

European Patent Office

Office européen des brevets

(19)

(11) Publication number: **0 154 351 B1**

## EUROPEAN PATENT SPECIFICATION

(12)

(45) Date of publication of patent specification: **14.08.91**

(51) Int. Cl.⁵: **C12N 15/59,** C12N 15/62, C12N 15/75, //(C12N9/56,9:64)

(21) Application number: **85102659.1**

(22) Date of filing: **08.03.85**

(54) Expression plasmids useful in B. subtilis.

(30) Priority: **09.03.84 JP 44900/84**

(43) Date of publication of application:
**11.09.85 Bulletin 85/37**

(45) Publication of the grant of the patent:
**14.08.91 Bulletin 91/33**

(84) Designated Contracting States:
**AT BE CH DE FR GB IT LI LU NL SE**

(56) References cited:
EP-A- 0 057 350     EP-A- 0 063 953
EP-A- 0 073 029     EP-A- 0 121 775
WO-A-85/00382     GB-A- 2 091 268

NATURE, vol. 293, no. 5832, October 1981, pp. 481-483; Macmillans Journals Ltd., Chesham Bucks, GB; K. HARDY et al.:"Production in B. subtilis of hepatitis B core antigen and of major antigen of foot and mouth disease virus"

(73) Proprietor: **Beppu, Teruhiko**
**No. 5-21, 1-chome**
**Horinouchi Suginami-ku, Tokyo(JP)**

(72) Inventor: **Beppu, Teruhiko**
**5-21, Horinouchi-1-chome**
**Suginami-ku Tokyo(JP)**
Inventor: **Uozumi, Takeshi**
**32-8 Takashimadaira-4-chome**
**Itabashi-ku Tokyo(JP)**
Inventor: **Tsuchiya, Makoto**
**4-9, Ikegami-5-chome**
**Yokosuka-shi(JP)**
Inventor: **Sekine, Susumu**
**E-22-406, 17, Tokiwadaira-4-chome**
**Matsudo-shi(JP)**

(74) Representative: **Henkel, Feiler, Hänzel & Partner**
**Möhlstrasse 37**
**W-8000 München 80(DE)**

EP 0 154 351 B1

J. BIOCHEM., vol. 90, no. 3, 1981, pp. 901-904; K. NISHIMORI et al.:"Cloning in Escherichia coli of the structural gene of prorennin, the precursor of calf milk-clotting enzyme rennin."

JOURNAL OF BACTERIOLOGY, vol. 150, no. 2, May 1982, pp. 804-814; S. HORINOUCHI et al.:"Nucleotide sequence and functional map of pE194, a plasmid that specifies inducible resistance to macrolide, lincosamide, and streptogramin type B antibiotics"

J.BIOCHEM., vol. 291, no. 3, 1982, pp. 1085-1088; K. NISHIMORI et al.:"Nucleotide sequence of calf prorennin cDNA cloned in Escherichia coli"

## Description

This invention relates to expression vectors useful in Bacillus subtilis. More particularly it relates to expression plasmids capable of expressing heterogeneous gene in B. subtilis under control of a $E_m r$ promoter, and to methods for expression of prochymosin cDNA in B. subtilis by means of said expression plasmids carrying the $E_m r$ promoter.

B. subtilis has been extensively studied from genetic and biochemical points of view, and is neither parasitic nor pathogenic to human or animal. Further, B. subtilis has a strong propensity to extracellularly secrete proteins produced. By this propensity it has become possible that Recombinant DNA technology is applied to have heterogeneous gene-derived products secreted extracellularly. Thus B. subtilis is considered as safe and valuable host organism when the Recombinant DNA Technology is tried on microorganism. However, as compared to E. coli, there are fewer successful instances wherein heterogeneous gene can indeed be expressed in B. subtilis as host organism: the major causes are the instability of plasmids within B. subtilis cells and the presence of strong proteolytic enzymes inherent in B. subtilis.

It is an objective of this invention to effect the heterogeneous gene expression in B. subtilis, and therefore to develop a new host-vector system for that purpose.

It is another objective of this invention to construct expression vectors capable of expressing genes in B. subtilis when the organism contain a promoter comprising an erythromycin-resistant ($E_m r$) gene.

It is a further objective of this invention to achieve the expression of prochymosin gene in B. subtilis by using the above-stated expression vectors.

The above described objectives and many other objectives as well as advantages associated with this invention will become clearer by reading the following description.

Prochymosin is secreted by the calf fourth stomach (abomasum), and is a precursor protein of chymosin which is responsible for coagulating milk in the manufacture of cheese. Prochymosin has a molecular weight of about 40,000 and consists of 365 amino acid residues. A peptide consisting of 42 amino acids is cleaved from the N-terminus of prochymosin by self-digestion, resulting in the formation of chymosin. Recently milk-coagulating enzymes, of microbial origin, have been discovered and these are substituting for chymosin.

Nevertheless, the need still exists for calf prochymosin in the cheese industry. Accordingly, it is one of the primary objectives in the application of Genetic Engineering to utilize the recombinant DNA technology and to have microorganisms produce the calf prochymosin.

Several successful instances have been reported wherein the recombinant DNA technology is applied to the production of prochymosin by E. coli host organism. For example, relevant references are: Nishimori et al., Gene, 19, 337 (1982); Japan Kokai 58-32,896 (to Teruhiko Beppu); Japan Kokai 57-141,287 (to Collaborative Research Inc.); and Japan Kokai 58-9,687 (to Celltech Ltd.).

Additionally, B. subtilis is disclosed as a host organism for production of the principle antigen for aphthous fever as well as for production of β-interferon, respectively in K, Hardy et al., Nature, 293, 481 (1981) and S. Chang et al., "Molecular Cloning and Gene Regulation in Bacilli", Academic Press, 159 (1982).

According to this invention there is provided a process for the production of prochymosin comprising the following steps:

a) inserting a prochymosin gene into a B. Subtilis vector system containing a promoter, with the proper reading frame, to form an expression plasmid;

b) transforming a B. subtilis host organism with the expression plasmid to obtain transformants;

c) selecting drug resistant transformant among the thus-obtained transformants;

d) growing said drug resistant transformant in a nutrient medium, and;

e) isolating prochymosin from the culture obtained in step (d).

In another aspect of this invention there are provided expression plasmids including an $E_m r$ promoter and a prochymosin cDNA sequence and capable of expressing prochymosin in host organism under control of the promoter.

Also included in this invention are methods for the construction of the novel expression plasmids, microorganisms transformed with said plasmids, and the prochymosin produced by the above-stated process.

In order to describe the invention fully, the description of preferred embodiments will follow.

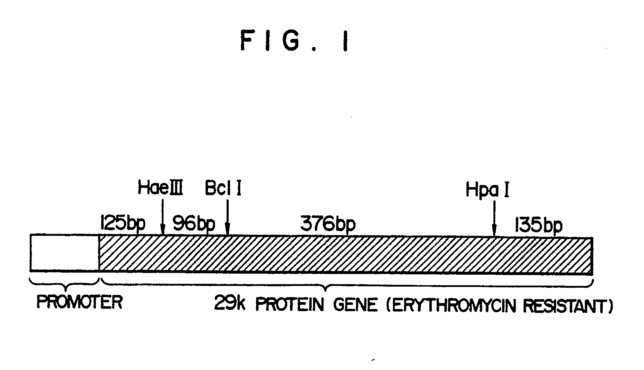

In the attached Drawings, figure 1 shows restriction enzyme map of 29K Protein.

Figure 2 shows a scheme for the construction of plasmid pKS3 from plasmids pHW1 and pMC1396, including the base sequence of pMC1396 around its Small site and of pHWl around its Haelll site.

Figure 3 shows a scheme for the construction of plasmid pCR702 from plasmids pCR701 and pBR322.

Figure 4 shows a scheme for the construction of plasmid pKSR101 from plasmids pCR702 and pKS3.

The Host Microorganisms

Wild-type species of B. subtilis demonstrate a defence mechanism against invading heterogeneous genes when introduced to their intracellular structure. Restriction enzymes capable of only cleaving the heterogeneous genes are responsible for that role. Thus, mutant strains deficient in such a restriction system are suitable as host organism.

Preferably, a Marbarg 168 derived strain of B. subtilis which is modified to be defective in the restriction system, B. subtilis RM125 (arg, leu, r⁻, m⁻), is used in this invention. See Uozumi et al., Molec. gen. Genet., 152, 65 (1977).

The Vectors

Suitable and useful vectors that are now commonly used to transform B. subtilis are three kinds of drug resistant plasmids derived from Staphylococcus; pUB110 ($k_m r$, 3.9Md), pE194 ($E_m r$, 2.4Md), and pC194 ($C_m r$, 2.0Md), respectively. In particular, the pE194 plasmid is valuable in the DNA manipulation, while considering the reading frame, since the entire base sequence of said plasmid is already known. In this invention the use of pHWI ($C_m r$, $E_m r$, 4.3Kb), which is one kind of pE194, is preferred. This particular plasmid is reported in S. Horinouchi, J. Bacteriol., 150, 8041 (1982).

The base sequence of the $E_m r$ promoter region within pHWI is, as indicated earlier, known and the promoter shows induction at a low concentration of $E_m$(erythromycin). The $E_m r$ gene in the plasmid codes for 23s RNA-methylation enzyme having a molecular weight of 29,000, which enzyme is synthesized inductively under control of the $E_m r$ promoter and will be hereinafter referred to as "29K Protein".

The Starting Plasmids

Plasmid pMC1396 ($A_p r$, lac'2Y, 9.9Kb) is described in M. J. Casadaban et al., J. Bacteriol., 143, 971 (1980), and plasmid pHWI ($C_m r$, $E_m r$, 4.3Kb) by Horinouchi (see above).

Plasmid pCR701 ($A_p r$, prochymosin, 5.34Kb) is described in N. Shimizu et al., Abstracts of the Annual Meeting of the Japanese Agricultural Chemistry Society, p.10 (1983), and has been integrated into a strain of E. coli, E. coli c600 $r_k^- m_k^-$ (pCR701), which is available as FERM BP264 deposited under the terms of the Budapest Treaty in the Fermentation Research Institute, Ibaragi, Japan.

Insertion of Heterogeneous Gene into the Restriction Enzyme Sites in 29K Protein $E_m r$ Gene

Plasmid pHWI contains three restriction enzyme sites within the 29K Protein gene (732bp); the HaeIII site 1256 bp downstream from the ATG start codon; the Bcl site 2216 bp downstream therefrom; and the HpaI site 597 bp downstream therefrom, respectively. Insertion of heterogeneous gene at any of these sites is possible. See Figure 1.

Plasmid pKS3

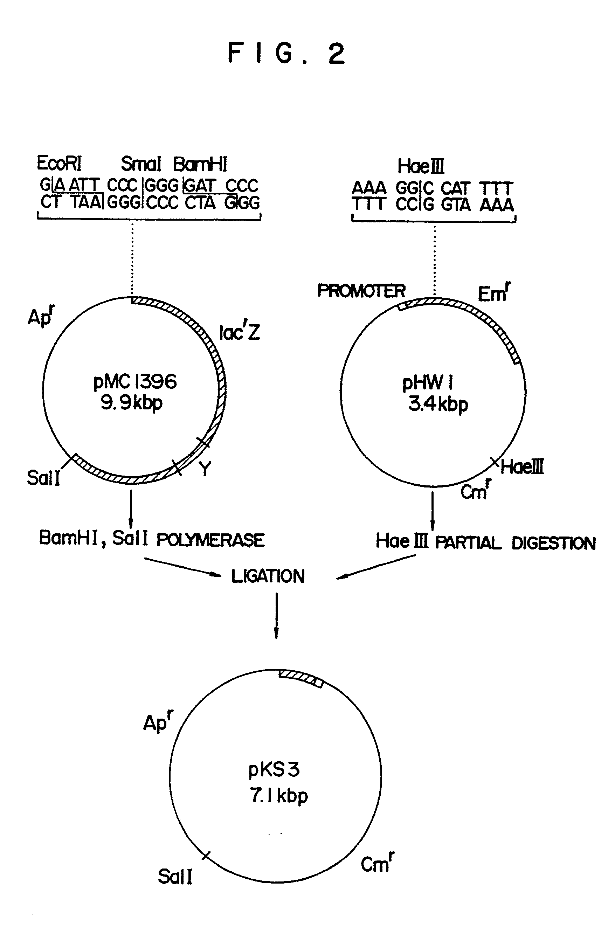

Expression plasmid pKS3 is constructed by integration at the HaeIII site in pHWI of an ampicillin-resistant gene portion (3.7Kb) that is flanked by the BamHI site and by the SalI site in pMC1396. First, the pMC 1396 is completely digested with BamHI and SalI. The linearized DNA is then treated with DNA polymerase (Klenow fragment) at its single-stranded portion, resulting in Fragment (a) (3.7Kb) with both blunt ends. The pHWI is partially digested with HaeIII which cleaves only HaeIII within the $E_m r$ gene. The thus produced Fragment (b) (3.4Kb) and Fragment (a) are ligated by means of $T_4$ DNA ligase. The ligation mixture is transformed into E. coli MC 1064 $r_k^- m_k^- s_m^r$ [M. J. Casadaban et al. J. Bacteriol., 143, 971 (1980)]. Transformants that are ampicillin and chloramphenicol resistant are selected on the basis of resistance to said reagents. The selected transformants were grown and harvested in a large preparation. Digestion with restriction endonucleases confirms that they carry pKS3. Figure 2 depicts the scheme for construction of pKS3 from pMC1396 and pHWI.

The base sequence in the vicinity of the HaeIII site in pK53 is shown:

```
G A A T T C C C G G G G A T C C C A T T T T
C T T A A G G G C C C C T A G G G T A A A A
       EcoRI    SmaI            BamHI
```

The ability of pKS3 to express heterogeneous genes in a B. subtilis host organism under control of the $E_m r$ promoter can be tested in the following manner. First, plasmid pKS3 is cleaved with SmaI and SalI, by which a $C_m r$ (chloramphenicol resistant) gene is isolated. Then, β-galactosidase structural gene is isolated from pMC 1403 ($A_p r$, lac'2Y, 9.9Kg) [M. J. Casadaban et al., J. Bacteriol., 143, 971 (1980)] by digestion with SmaI and SalI. Both digests are ligated by means of $T_4$ DNA ligase and the thus produced mixture is transformed into the host B. subtilis RM 125. Transformants are grown in L Broth containing chloramphenicol, erythromycin, and Xgal (5-bromo-4-chloro-3-indolyl-β-D-galactoside) available from Sigma Co. Ltd. If blue colonies are detected in the culture, then it will be clear that β-galactosidase is produced by the transformants. The pKS3 can then express β-galactosidase in the B. subtilis host organism

Similarly, it is possible to insert into the BclI Site in the 29K Protein gene, the β-galactosidase structural gene from pMC1403. As described for pKS3, the resulting plasmid can be tested as to whether or not it can express β-galactosidase in the B. subtilis host cells. The same applies to the expression plasmid wherein the HpaI site of the 29K Protein gene is utilized as receiving site of the β-galactosidase gene. Its ability to express β-galactosidase can also be tested accordingly. Based on the results of expression capabilities of β-galactosidase, the highest expression site can be chosen and used for construction of expression vectors including desired heterogeneous genes.

Not only the pKS3 but also other expression vectors derivable from pHWI that carries the β-galactosidase insert at its BclI or HpaI site do express said inserted gene; however, the expression levels of the latter vectors are much lower than that of pKS3.

Plasmid pCR702

The plasmid pCR701 carries the full-length cDNA of prochymosin. This prochymosin cDNA is, therefore, available from pCR701 by digestion with HindIII and SalI. The cut DNA must, however, contain a poly dG (poly dc) sequence. Since this poly dG sequence lowers the transformation efficiency in B. subtilis, it will be necessary to remove said sequence when the prochymosin DNA is integrated into a B. subtilis expression vector. For this reason, the pCR701 is completely digested with HinfI, and then with nuclease SI to remove the single-stranded portion. A SalI linker (GGTCGACC) is linked to this fragment consisting of 2550 bp and it is further digested with SalI to isolate fragment(c).

Plasmid pBR322 is completely digested with SalI, and then treated with Calf Alkaline Phosphatase to isolate fragment(d). Fragment(c) and fragment(d) are ligated by means of $T_4$DNA ligase. This ligation mixture is transformed into E. coli MC1064$r_k^- m_k^+ s_m^r$. Transformants are selected based on the ampicillin and tetracycline resistance. These transformant colonies are cultured on a small scale, and plasmid DNA are subjected to restriction enzyme analysis, which verify the presence of pCR702 (5.3Kb).

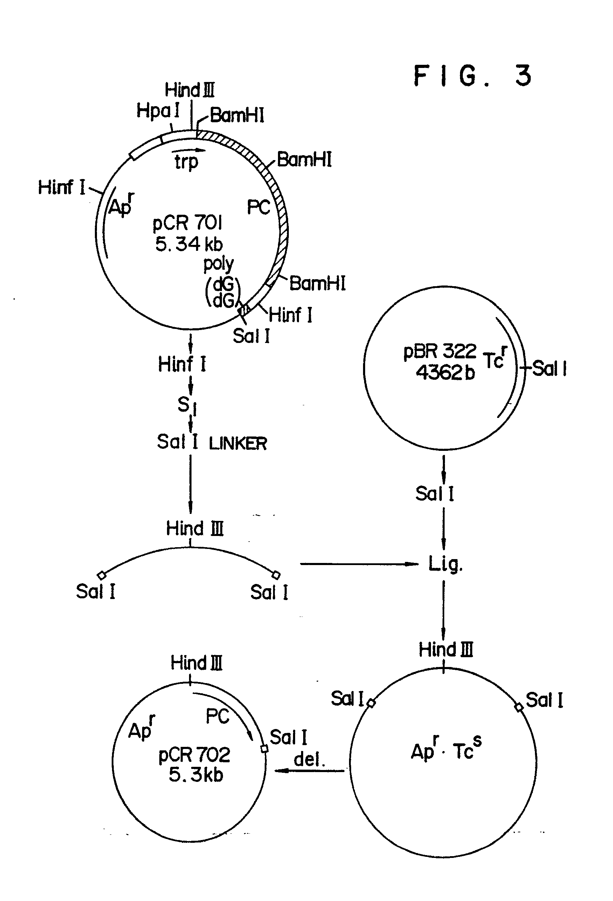

The scheme used for constructing pCR702 from pCR701 and pBR322 is illustrated in Figure 3. It will be recognized that pCR702 is devoid of the SalI-Hinf part of pCR701. Transformants carrying pCR702 produces prochymosin.

Plasmid pKSR101

The plasmid pKSR101 has the $E_m r$ promoter adjacent to the full-length prochymosin cDNA in the proper reading frame. Construction of pKSR101 is carried out in the following manner. First, pKS3 is doubly digested with BamHI and SalI to isolate DNA fragment(e) containing $C_m^r$ part of 4.3 Kb. This fragment includes the $E_m^r$ promoter and a part of the 29K Protein gene. Plasmid pCR702 is completely digested with BamHI and EcoRI to isolate DNA fragment(f) consisting of 465 bp. Further, pCR702 is doubly digested with EcoRI and SalI to isolate DNA fragment(g). Fragments(a) and (b) are both prochymosin cDNAs, and together they will make nearly the full-size prochymosin gene. Fragments (e), (f), and (g) are ligated together by means of $T_4$ ligase. This ligated DNA is transformed into B. subtilis. Among transformants produced, chloramphenicol resistant colonies are picked, and are found to contain pKSR101 as determined by restriction enzyme analysis.

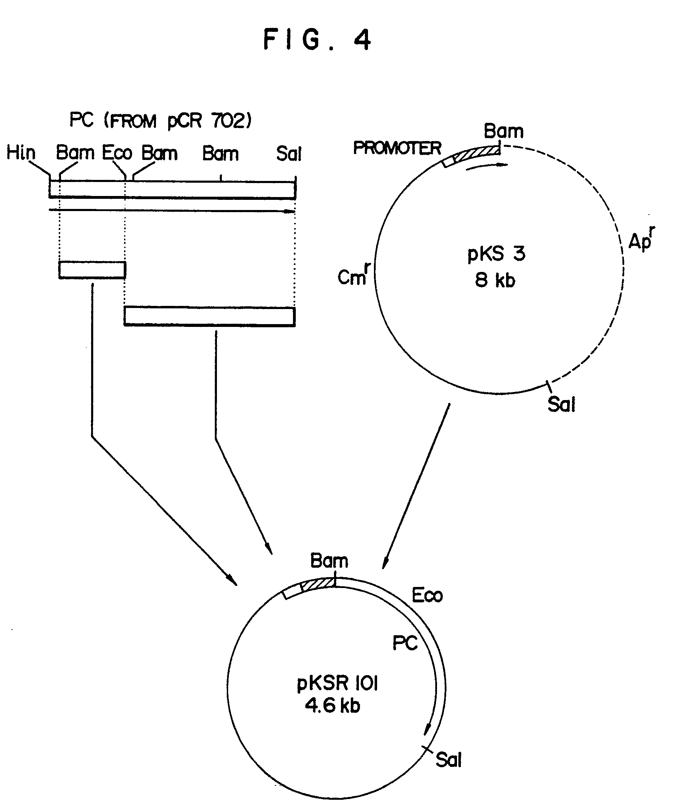

The scheme used for constructing pKSR101 from pCR702 and pKS3 is illustrated in Figure 4. The DNA sequence around the fifth prochymosin codon (CCT) has been shown by the Maxam-Gilbert method to be as follows:

```
        5'       BamHI                       3'

        -A  A  A  G  G|G  A  T  C  C  C  T  C  T  G-

         T  T  T  C  C  C  T  A  G|G  G  A  G  A  C

         29K                          Arg

                                   Prochymosin
```

Expression of Prochymosin in B. subtilis

After B. subtilis transformant cells carrying plasmid pKSR101 have preliminarily been grown in LB broth, cultures are cultivated fully in CH medium. During the latter culturing erythromycin is added to induce gene expression. Cells are collected from the fermentation culture and the cell protein extract is subjected to SDS polyacrylamide gel electrophoresis. If the migrated proteins are blotted onto nitrocellulose filters and are analyzed by the enzyme immunoblotting assay, the prochymosin protein can be detected. The production level of protein is estimated to be around 75,000 to 15,000 molecules per cell, and this quantitation is based on by calibrating the authentic prochymosin. The product protein is in fact a fused protein comprising prochymosin and the 29K protein, and the estimated molecular weight of such a polypeptide is about 45,000. This observation implies that expression in B. subtilis RM125 (pKSR101) of the prochymosin protein fused to 29K Protein is under control of the $E_m$r promoter and that the 29K protein is resulted from the 29K Protein gene which is downstream from the start codon and intervenes between the prochymosin gene and the start codon.

E. coli strains or B. subtilis strains produced in this invention, which contain novel expression plasmids, have been deposited in the Fermentation Research Institute, the Agency of Industial Science and Technology, Ibaragi, Japan, under the terms of the Budapest Treaty. They are identified by the accession numbers given thereto.

This invention will be described in some more detail by way of illustration and example; nevertheless, it should be understood that certain changes and modifications be practiced within the scope of the invention.

EXAMPLE

Several kinds of buffered solutions that are used throughout the following examples have compositions as tabulated below. Unless otherwise stated, the TEN buffered solution is employed.

| TE buffer | TEN buffer |
|---|---|
| 10mM Tris-HCl | 20mM Tris-HCl |
| (pH 8.0) | (pH 7.5) |
| 1mM EDTA | 50mM NaCl |
| | 1mM EDTA |

| (x10) Nuclease | (x10) DNA Polymerase |
|---|---|
| S1 buffer | buffer |
| 0.3M Na-Acetate | 0.3M Tris-HCl |
| (pH 4.6) | (pH 7.5) |
| 0.5M NaCl | 40mM $MgCl_2$ |
| 10mM $ZnSO_4$ | 5mM β-mercaptoethanol |
| $T_4$ DNA ligase buffer | $T_4$ DNA kinase buffer |
| 66mM Tris-HCl (pH 7.6) | 500mM Tris-HCl (pH 7.5) |
| 66mM $MgCl_2$ | 100mM $MgCl_2$ |
| 10mM DTT | 50mM DTT |
| | 1mM Spermidine |
| | 1mM EDTA |

Buffered Solution for Use in Gel Electrophoresis
40mM Tris-HCl (pH 7.5)
5 mM $CH_3COONa$
1 mM EDTA

Nutrient media used for transformation and expression experiments are:

L-Broth 1 l (pH 7.2-7.4)

| | |
|---|---|
| Bacto-Trypton | 10g |
| Yeast Extract | 5g |
| Glucose | 1g |
| NaCl | 5g |

CH Broth 1 l (pH 7.0)

| | |
|---|---|
| $K_2HPO_4$ | 14 g |
| $KH_2PO_4$ | 6 g |

| Na$_3$-citrate | 1 g |
| (NH$_4$)$_2$SO$_4$ | 2 g |
| Difco Casein Hydrolysate | 0.5g |
| MgSO$_4$.7H$_2$O | 0.2g |
| adenine | 100mg |
| lysine | 50mg |

The method of Norgard [M.V. Norgard et al., Gene, 3, 279 (1978)] is adapted for E. coli transformation, and the method of Spizizen [J. Spizizen et al., J. Bacteriol., 81, 741 (1962)] for B. subtilis transformation.

### E. coli Transformation

E. coli cells are grown in LB broth (10 ml) to densities of 1 to 2 x 10$^3$ cells/ml. The cultured cells are harvested and washed with 10 ml of pCPII. Once again, cells are harvested and suspended in 5 ml PCPII and the suspension is allowed to stand for 20 min. They are reharvested and resuspended in 0.5ml PCPII and competent cells (i.e., cells capable of accepting foreign DNA) are obtained. A transforming DNA insert is added to 0.5 ml competent cell suspension and this is allowed to stand at 0°c for 30-60 min. To the culture 1.8 ml of L broth is added and this is shaken at 37°c for 1.5 h. The resulting cultures (0.1 ml) are inocculated on plates containing a drug such as ampicillin, and drug resistant transformants are sellected.

### B. subtilis Transformation

B. subtilis cells are preliminarily grown in L broth. One tenth of the culture (10 ml) is inocculated on MI (2 ml), and incubated under shaking at 37°c for 4 h. Then one tenth of the resulting culture is inocculated on MII and incubated at 37°c for 1.5 h. The thus obtained competent cells are kept frozen [D. Dubnau et al., J. Virol., 4, 50 (1969)]. To these frozen competent cells (0.3 ml) is added EGTA to make up a final concentraiton of 1mM EGTA. After the solution is preincubated at 37°c for 5 min, a transforming DNA solution is added and incubated at 37°c for 45 min. One or two volumes of LB broth are added to she incubated mixture and it is kept at 37°c for 60 min to effect expression. This culture (0.1 ml) is then inocculated on plates containing a drug, and drug resistant transformants are selected.

### EXAMPLE 1

### Construction of pKS3

i) Partial Digestion of pHW (C$_m$r, E$_m$r, 3.4Kb) with HaeIII

Plasmid pHW1 was partially digested with HaeIII by incubation at 37°c for 2 min in a 40 $\mu$l solution containing TE Plasmid solution 20 $\mu$l (50 $\mu$g/ml), HaeIII (5U, 1 $\mu$l), x10 HaeIII buffer (3 $\mu$l) and H$_2$O (6 $\mu$l). The digestion was stopped by phenol treatment, and the resulting DNA (b) was precipitated with ethanol. The DNA was dissolved in 6 $\mu$l of H$_2$O and this dissolved solution was used in the ligation step IV).

ii) Complete Digestion of pMC1396 (A$_p$r, lac'z-y, 9.9Kb) with BamHI and SalI

A mixture of Plasmid pMC1396 TE solution 20 $\mu$l (52 $\mu$g/ml), BamHI (30U, 3 $\mu$l), and X10 BamHI buffer solution 3 $\mu$l was incubated at 37°c for 1 h. The DNA was precipitated with ethanol, and the precipitate was dissolved in 24 $\mu$l H$_2$O. To this dissolved solution was added SalI (30U, 3$\mu$l) and X10 SalI buffer 3 $\mu$l to make up a 30 $\mu$l solution and it was incubated at 37°c for 1 h followed by incubation at 65°c for 10 min. The DNA(a) was precipitated with ethanol.

iii) Repairing of the Single Stranded End in DNA Fragment (a)

The DNA(a) was dissolved in 29 $\mu$l H$_2$O and to this was added 20mM dNTP (each 2.5 $\mu$l; total 10 $\mu$l), polymerase buffer 5 $\mu$l, and Klenow fragment of DNA polymerase I (1.5U, 1 $\mu$l) to make up a 45 $\mu$l mixture. It was incubated at 30°c for 10 min. The reaction was terminated by phenol treatment. The

EP 0 154 351 B1

resulting DNA was precipipated with ethanol and dissolved in 6 $\mu$l $H_2O$. This dissolved solution was used in the next step.

iv) Ligation of DNA Fragment (a) with DNA Fragment (b)

A mixture (total volume of 16 $\mu$l) of each of 6 $\mu$l of DNA (a) and DNA (b) solutions, $T_4$ ligase (1.8U, 2 $\mu$l), 3mMATP (4 $\mu$l), and X10 ligase buffer (2 $\mu$l) was incubated at 11$^\circ$c overnight. The ligated DNA was precipitated with ethanol.

v) Transformation

The DNA was introduced by transformation into competent cells of E. coli MC1064 ($r_k^- m_k^+ sm^r$) that were prepared according to the conventional procedure. Among transformants produced, ampicillin and chloramphenicol resistant colonies were selected. Plasmid DNA was extracted from the drug resistant colonies. Restriction endonuclease analysis of the plasmid showed that these colonies contain plasmid pKS3. The strain, designated as E. coli MC 1064 $r_k^- m_k^+ sm^r$(pKS3), was deposited under the terms of the Budapest Treaty in the FRI with Accession NO. FERM BP-503.

EXAMPLE 2

Construction of pCR702

i) Digestion of pCR701 with Hinfl

Plasmid pCR701 was completely digested with Hinfl by incubation at 37$^\circ$c for 1.5 h in a 100 $\mu$l mixture containing Plasmid 65 $\mu$l (150 $\mu$g/ml), Hinfl (35U, 7 $\mu$l), x10 Hinf buffer 10 $\mu$l, and $H_2O$ 18 $\mu$l. The resultant DNA was precipitated with ethanol. The precipitate was dissolved in $H_2O$ (263.7 $\mu$l) and this was used in the next step.

ii) Nuclease SI Treatment

A mixture (total of 300 $\mu$l) of the DNA solution 263.7 $\mu$l, Nuclease SI and X10 Nuclease SI buffer 30 $\mu$l was incubated at 40$^\circ$c for 30 min. The reaction was terminated by phenol treatment. Phenol was removed by ether extraction. The DNA was precipitated with ethanol. It was then electrophoresed on a 0.8 % agarose gel and the largest DNA fragment (2550 bp) was isolated by electroelution.

iii) Ligation of Sall Linker to the DNA Fragment

Mixture A was prepared by mixing the DNA fragment (8 $\mu$l $H_2O$), $H_2O$ 4 $\mu$l and X5 ligase buffer 1 $\mu$l. Next, Mixture B was prepared by mixing Sall linker (GGTCGACC) 3 $\mu$l(1 $\mu$g/$\mu$l), X10 potassium phosphate buffer 3$\mu$l, 3mM ATP 3 $\mu$l, $H_2O$ 19 $\mu$l, and $T_4$ DNA kinase (6U, 2 $\mu$l) and incubating at 37$^\circ$c for 1 h. After mixure (A) and mixture (B) were mixed, 3mMATP 5 $\mu$l and $T_4$ ligase 3 $\mu$l (1.8 $\mu$) were added and the reaction mixture was incubated at 11$^\circ$c overnight, followed by incubation at 65$^\circ$c for 10 min.

iv) Digestion with Sall

To the reaction obtained in step iii) were added Sall (200U, 10 $\mu$l), $H_2O$ 117 $\mu$l, and X10 Sall buffer 20 $\mu$l, and the mixture was incubated at 37$^\circ$c for 2 h. Further, to the mixture were added Sall (200U, 20 $\mu$l), X10 Sall buffer 20 $\mu$l, and $H_2O$ 160 $\mu$l and the whole mixture was incubated at 37$^\circ$c for 2 h, completing digestion with Sall. The resultant DNA was precipitated with ethanol, and electrophoresed on a 0.8 % agarose gel to remove the remaining linker.

v) Digestion of pBR322 with Sall and Treatment thereof with Alkaline Phosphatase

Plasmid pBR322 was digested with Sall by incubation at 37$^\circ$c for 1 h in a 70 $\mu$l solution containing Plasmid 20 $\mu$l (259 $\mu$g/ml), Sall (70U, 7 $\mu$l), X10 Sall buffer 7 $\mu$l, and $H_2O$ 36 $\mu$l. The resulting DNA was precipitated with ethanol. The precipitate was dissolved in 78 $\mu$l $H_2O$. To this DNA solution were added X10 DP buffer 9 $\mu$l which contains 100 mM $MgCl_2$ in 0.5M Tris-HCl (pH 8.5) and Calf intestine alkaline phosphatase (6U, 3 $\mu$l). The mixture was incubated at 37$^\circ$c for 1 h. Reaction was terminated by phenol treatment.

vi) Religation

DNA (c) obtained in step (iv) was dissolved in 40 $\mu$l $H_2O$. Twenty $\mu$l was used in ligation. DNA (d) obtained in step (v) was dissolved in 20 $\mu$l $H_2O$. Five $\mu$l was used in ligation. Both DNA solutions were mixed. To this were added 3m MATP 7 $\mu$l, $T_4$ ligase (0.9U, 1 $\mu$l), and X10 ligase buffer and the mixture was incubated at 21$^\circ$c for 2h to complete ligation. The ligated DNA was precipitated with ethanol. It was recognized that this ligation process caused deletion of the overlapping part from pBR322 in the resulting DNA plasmid.

vii) Transformation

The DNA was introduced by transformation into competent cells of E. coli MC1064 ($r_k^- m_k^+ s_m^r$) that were prepared according to the conventional procedure. Among about 500 colonies, about 70 ampicillin and tetracycline resistant colonies were selected. Plasmid DNA was extracted from the drug resistant

9

colonies. Restriction endonuclease analysis of the plasmid showed that these colonies contain plasmid pCR702. The strain, designated as E. coli MC1064 $r_k^- m_k^+ s_m^r$ (pCR702), was deposited under the terms of the Budapest Treaty in the FRI with Accession No. FERM BP-501.

EXAMPLE 3

Construction of Plasmid pKSR101

i) Complete Digestion of pKS3 with BamHI and SalI

Plasmid pKS3 was completely digested with SalI by incubation at 37°c for 1 h in a 55 $\mu$l solution containing Plasmid 30 $\mu$l (50 $\mu$g/ml), SalI (50U, 5 $\mu$l), SalI buffer 5 $\mu$l, and $H_2O$ (15 $\mu$l). The resultant DNA was precipitated with ethanol. The precipitate was dissolved in 40 $\mu$l $H_2O$. To this dissolved solution were added BamHI (50U, 5 $\mu$l) and X10 BamHI buffer 5 $\mu$l. The mixed solution was incubated at 37°c for 1 h. The resultant DNA was precipitated with ethanol, and was electrophoresed on an agarose gel and the largest DNA fragment (e) (4.3 Kb) was isolated by electroelution.

ii) Double Digestion of pCR702 with BamHI and EcoRI

Plasmid pCR702 was digested with EcoRI by incubation at 37°c for 1 h in a 50 $\mu$l solution containing Plasmid 20 $\mu$l(150 $\mu$g/ml), EcoRI (75U, 15 $\mu$l), and X10 EcoRI buffer 5 $\mu$l, and $H_2O$ 20 $\mu$l. To this solution were added BamHI (50U, 5 $\mu$l), BamH buffer 5 $\mu$l to make up a 50 $\mu$l solution. The mixed solution was incubated at 37°c for 1 h. The cut DNA was precipitated with ethanol, and electrophoresed on an agarose gel and the smallest fragment (f) (465bp) was isolated.

iii) Double Digestion of pCR702 with EcoRI and SalI

Plasmid pCR702 was digested with SalI by incubation at 37°c for 1 h in a 50 $\mu$l solution contaiing Plasmid 30 $\mu$l (130 $\mu$g/ml), SalI (50U, 5 $\mu$l), X10 SalI buffer (5 $\mu$l), and $H_2$ (10 $\mu$l). The resultant DNA was precipitated with ethanol. The precipitate was dissolved in 40 $\mu$l $H_2O$. To this dissolved solution were added EcoRI (75U, 5 $\mu$l) and EcoRI buffer (5 $\mu$l) to make up a 50 $\mu$l solution. The solution was incubated at 37°c for 1 h. The cut DNA was precipitated with ethanol, and electrophoresed on an agarose gel to isolate the smallest fragment (g) (729 bp).

iv) Ligation of DNA Fragments (e), (f) and (g)

DNA fragment (e) (4.3 Kb), fragment (f) (4646 bp), fragment (g) (729bp) were dissolved in 18 $\mu$l $H_2O$. To this DNA solution were added $T_4$ ligase (2.7U, 3 $\mu$l) 3mMATP (6 $\mu$l), X10 ligase buffer (3 $\mu$l) to make up a 30 $\mu$l solution. The solution was incubated at 11°c overnight. The ligated DNA was precipitated with ethanol.

v) Transformation

The DNA was introduced by transformation into competent cells of B. subtilis RM125 (arg, leu, r⁻, m⁻) that were prepared according to the conventional procedure. Among transformants, two chloramphenicol resistant colonies were selcted, from which plasmid DNA was extracted. Restriction endonuclease analysis of the plasmid showed that these colonies contain plasmid pKSR101. The strain, designated as B. subtilis RM125 (arg, leu, r⁻, m⁻; pKSR101), was deposited under the terms of the Budapest Treaty in the FRI with Accession No. FERM BP-504.

EXAMPLE 4

Expression of Prochymosin in B. subtilis

B. subtilis RM 125 carrying pKSR101 was grown in LB broth containing 10 $\mu$g/ml of chloramphenicol. Cells were grown in shaker flasks at 37°c overnight. A 0.2 ml culture was inoculated onto CH broth and was incubated at 37°c under shaking. When the cell density reached at about 3 x 10⁷ cells per ml, erythromycin was added (final concentration of 0.05 $\mu$g/ml) and cultivation was continued for another hour. One ml of the culture was taken and cells were harvested and washed with cold PBS (20mM phosphate buffer, 150mM NaCl, pH 7.0). Cells were collected again. The cells were then added to 7 $\mu$l of lysing buffer (30mM Tris-HCl, 5mM EDTA, 50mM NaCl, 2mM PMSF, pH 8.0) that contains lyozyme (1 mg/ml). Incubation continued at 37°c for 10 min. Further, 7 $\mu$l of x4 Sample buffer (0.0625M Tris-HCl, 2% SDS, 10% glycerol, 5% $\beta$-mercaptoethanol, pH 6.8) was added to the above incubated solution, and this was heated at 100°c for 10 min. After addition of 1 to 2 $\mu$l of PYRONINE G solution (10 mg/ml ETOH), it was electrophoresed on 10% SDS-polyacrylamide gels. The migrated proteins were blotted onto nitrocellulose filters according to the electroblotting method [B. Bowen et al., Nucleic Acid Res., 8, 1 (1980)] using TRANS-BLOT CELL. These nitrocellulose filters were colored and proteins were assayed by BIO-RAD BLOT ASSAY KIT using Goat Anti-Rabbit IgG Horseradish Peroxidase Conjugate): the KIT was purchased from BIORAD Co., Ltd. This analysis (enzyme immunoblotting assay) established that B. subtilis RM 125 (pKSR101) produced the prochymosin-fused protein. In an effort to determine the level of protein expression, the band of the protein produced was compared in intensity with those of varying concentrations of the authentic prochymosin. The intensity of the product protein band was determined to be in the range of from 50 ng to 100 ng of prochymosin. Accordingly, the level of production of the prochymosin-fused protein was about 7,500 - 15,000 molecules per host cell. The estimated molecular weight of the product protein was about 45,000 as measured by using a molecular marker, which value was well in accord with the calculated value assuming that the product protein would be a fused protein of prochymosin and 29K Protein.

**Claims**

1. A process for producing prochymosin which process comprises:
   a) transforming a B. subtilis host organism with the expression plasmid pKSR101 as found in B. subtilis deposited in the FRI as FERM BP-504;
   b) selecting a chloramphenicol resistant transformant or transformants obtained in step (a);
   c) cultivating said chloramphenicol resistant transformant or transformants in a nutrient medium; and
   d) isolating said prochymosin.

2. A process according to claim 1 wherein said B. subtilis host organism is B. subtilis RM 125 (arg, leu, r , m ) as deposited in the FRI as FERM BP-504.

3. A process for producing prochymosin, which process comprises:
   a) cultivating, in a nutrient medium , a B. subtilis transformant comprising the expression plasmid pKSR101 as found in B. subtilis deposited in the FRI as FERM BP-504; and
   b) isolating said prochymosin.

4. A process according to claim 3 wherein said B. subtilis transformant is the strain deposited in the FRI as FERM BP-504.

5. An expression plasmid which comprises a $E_m r$ promoter and a prochymosin cDNA and is capable of expressing prochymosin in B. subtilis under control of said promoter, said expression plasmid being pKSR101.

6. An expression plasmid according to claim 5 wherein said B. subtilis organism is B. subtilis RM 125 (arg, leu, $r^-$, $m^-$).

7. A process for preparing plasmid pKS3 which comprises the steps of
   a) partially digesting plasmid pHWI with HaeIII to obtain the blunt ended DNA fragment (1);
   b) digesting plasmid pMC 1396 with BamHI and SalI;
   c) repairing by means of DNA polymerase the single stranded portion of the DNA obtained in step (b) to form DNA fragment (2); and
   d) ligating DNA fragments (1) and (2) by $T_4$ ligase to obtain plasmid pKS3.

8. A process for preparing plasmid pCR702 which comprises the steps of
   a) digesting plasmid pCR701 as found in E. coli deposited in FRI as FERM BP-264 with HinfI, followed by cutting the single stranded DNA with nuclease SI to obtain DNA fragment (3);
   b) attaching SalI linker to DNA fragment (3) and thereafter digesting it with SalI to obtain DNA fragment (4);
   c) digesting plasmid pBR322 with SalI to obtain DNA fragment (5); and
   d) ligating DNA fragments (4) and (5) by means of $T_4$ ligase to obtain plasmid pCR702.

9. A process for preparing plasmid pKSR101 which comprises the steps of
   a) digesting plasmid pKS3 as found in E. coli deposited in FRI as FERM BP-503 with BamHI and SalI to obtain DNA fragment (6);
   b) completely digesting plasmid pCR702 as found in E. coli deposited in FRI as FERM BP-501 with BamHI and EcoRI to obtain DNA fragment (7);
   c) digesting plasmid pCR702 as found in E. coli deposited in FRI as FERM BP-501 with EcoRI and

EP 0 154 351 B1

Sall ot obtain DNA fragment (8); and
d) ligating DNA fragments (6), (7) and (8) by means of T₄ ligase to obtain plasmid pKSR 101.

**10.** Plasmid pKS3 found in E. coli deposited in the FRI as FERM BP-503.

**11.** Plasmid pCR702 found in E. coli deposited in the FRI as FERM BP-501.

**12.** Plasmid pKSR101 found in B. subtilis deposited in the FRI AS FERM BP-504.

**13.** E. coli MC 1064 $r_k^-m_k^+s_m^r$ (pKS3) deposited in the FRI as FERM BP-503.

**14.** E. coli MC1064 $r_k^-m_k^+s_m^r$ (pCR702) deposited in the FRI as FERM BP-501.

**15.** B. subtilis RM125 $r^-m^-$, arg, leu (pKSR101) deposited in the FRI as FERM BP-504.

## Revendications

**1.** Procédé de préparation de prochymosine, comprenant les étapes consistant
a) à transformer un organisme hôte B. subtilis avec le plasmide d'expression pKSR101 que l'on trouve dans le B. subtilis déposé au FRI sous le numéro FERM BP-504;
b) à sélectionner un ou des transformants résistants au chloramphénicol parmi ceux qui ont été obtenus dans l'étape (a);
c) à cultiver ledit ou lesdits transformants résistants au chloramphénicol dans un milieu nutritif; et
d) à isoler ladite prochymosine.

**2.** Procédé selon la revendication 1, dans lequel ledit organisme hôte B. subtilis est le B. subtilis RM 125 (arg, leu, $r^-$, $m^-$), déposé au FRI sous le numéro FERM BP-504.

**3.** Procédé de préparation de prochymosine, comprenant les étapes consistant
a) à cultiver, dans un milieu nutritif, un transformant de B. subtilis comprenant le plasmide d'expression pKSR101 que l'on trouve dans le B. subtilis déposé au FRI sous le numéro FERM BP-504; et
b) à isoler ladite prochymosine.

**4.** Procédé selon la revendication 3, dans lequel ledit transformant de B. subtilis est la souche déposée au FRI sous le numéro FERM BP-504.

**5.** Plasmide d'expression qui comprend un promoteur $E_m^r$ et un ADNc de prochymosine et qui est capable d'exprimer la prochymosine dans B. subtilis sous le contrôle dudit promoteur, ce plasmide d'expression étant le pKSR101.

**6.** Plasmide d'expression selon la revendication 5, ledit organisme B. subtilis étant le B. subtilis RM 125 (arg, leu, $r^-$, $m^-$).

**7.** Procédé de préparation du plasmide pKS3, comprenant les étapes consistant
a) à digérer partiellement le plasmide pHWI avec HaeIII pour obtenir le fragment d'ADN à extrémités droites (1);
b) à digérer le plasmide pMC 1396 avec BamHI et SalI;
c) à réparer, au moyen d'ADN-polymérase, la partie à un seul brin de l'ADN obtenu dans l'étape (b) pour former un fragment d'ADN (2); et
d) à lier les fragments d'ADN (1) et (2) par la T₄-ligase pour obtenir le plasmide pKS3.

**8.** Procédé de préparation du plasmide pCR702, comprenant les étapes consistant
a) à digérer avec HinfI le plasmide pCR701 que l'on trouve dans l'E. coli déposé au FRI sous le numéro FERM BP-264, puis à couper l'ADN à un seul brin avec la nucléase SI pour obtenir un fragment d'ADN (3);
b) à attacher le segment de liaison SalI au fragment d'ADN (3), puis à le faire digérer avec SalI pour obtenir un fragment d'ADN (4);

12

c) à digérer le plasmide pBR322 avec SalI pour obtenir un fragment d'ADN (5); et

d) à lier les fragments d'ADN (4) et (5) au moyen de $T_4$-ligase pour obtenir le plasmide pCR702.

9. Procédé de préparation du plasmide pKSR101, comprenant les étapes consistant

a) à digérer, avec BamHI et SalI, le plasmide pKS3 que l'on trouve dans l'*E. coli* déposé au FRI sous le numéro FERM BP-503, pour obtenir un fragment d'ADN (6);

b) à digérer complètement, avec BamHI et EcoRI, le plasmide pCR702 que l'on trouve dans l'*E. coli* déposé au FRI sous le numéro FERM BP-501, pour obtenir un fragment d'ADN (7);

c) à digérer, avec EcoRI et SalI, le plasmide pCR702 que l'on trouve dans l'*E. coli* déposé au FRI sous le numéro FERM BP-501, pour obtenir un fragment d'ADN (8); et

d) à lier les fragments d'ADN (6), (7) et (8) au moyen de $T_4$-ligase pour obtenir le plasmide pKSR101.

10. Plasmide pKS3 que l'on trouve dans l'*E. coli* déposé au FRI sous le numéro FERM BP-503.

11. Plasmide pCR702 que l'on trouve dans l'*E. coli* déposé au FRI sous le numéro FERM BP-501.

12. Plasmide pKSR101 que l'on trouve dans le *B. subtilis* déposé au FRI sous le numéro FERM BP-504.

13. *E. coli* MC 1064 $r_k^- m_k^+ s_m^r$ (pKS3) déposé au FRI sous le numéro FERM BP-503.

14. *E. coli* MC 1064 $r_k^- m_k^+ s_m^r$ (pCR702) déposé au FRI sous le numéro FERM BP-501.

15. *B. subtilis* RM125 $r^- m^-$, arg, leu (pKSR101) déposé au FRI sous le numéro BP-504.

**Patentansprüche**

1. Verfahren zur Herstellung von Prochymosin durch

a) Transformieren eines B. subtilis-Wirtorganismus mit dem in B. subtilis gefundenen Expressionsplasmid pKSR101 (hinterlegt bei der FRI unter der Hinterlegungsnummer FERM BP-504);

b) Selektieren eines (von) in Stufe a) erhaltenen, chloramphenicolresistenten Transformanten;

c) Wachsenlassen des (der) chloramphenicolresistenten Transformanten in einem Nährmedium und

d) Isolieren des Prochymosins.

2. Verfahren nach Anspruch 1, dadurch gekennzeichnet, daß es sich bei dem B. subtilis-Wirtorganismus um bei der FRI unter der Hinterlegungsnummer FERM BP-504 hinterlegtes B. subtilis RM 125 (arg, leu, r , m ) handelt.

3. Verfahren zur Herstellung von Prochymosin durch

a) Wachsenlassen eines B. subtilis-Transformanten mit dem in bei der FRI unter der Hinterlegungsnummer FERM BP-504 hinterlegten B. subtilis gefundenen Expressionsplasmid pKSR101 in einem Nährmedium und

b) Isolieren des Prochymosins.

4. Verfahren nach Anspruch 3, dadurch gekennzeichnet, daß es sich bei dem B. subtilis-Transformanten um den bei der FRI unter der Hinterlegungsnummer FERM BP-504 hinterlegten Stamm handelt.

5. Expressionsplasmid mit einem $E_m r$-Promotor und einer Prochymosin-cDNA und der Fähigkeit zur Expression von Prochymosin in B. subtilis unter Steuerung des betreffenden Promotors, das als pKSR101 bezeichnet ist.

6. Expressionsplasmid nach Anspruch 5, dadurch gekennzeichnet, daß es sich bei dem B. subtilis-Organismus um B. subtilis RM 125 (arg, leu, $r^-$, $m^-$) handelt.

7. Verfahren zur Herstellung des Plasmids pKS3 in folgenden Stufen:

a) teilweises Verdauen des Plasmids pHWI mit HaeIII zur Bildung eines stumpfendigen DNA-Fragments (1);

b) Verdauen des Plasmids pMC 1396 mit BamHI und SalI;

c) Reparieren des einstrangigen Teils der in Stufe (b) erhaltenen DNA mittels DNA-Polymerase zur Bildung eines DNA-Fragments (2) und

d) Verbinden der DNA-Fragmente (1) und (2) mittels T₄-Ligase zur Gewinnung des Plasmids pKS3.

8. Verfahren zur Herstellung des Plasmids pCR702 in folgenden Stufen:

a) Verdauen des in E. coli aufgefundenen Plasmids pCR701 (hinterlegt bei der FRI unter der Hinterlegungsnummer FERM BP-264) mit Hinfl und anschließendes Schneiden der einstrangigen DNA mit Nuklease SI zur Bildung eines DNA-Fragments (3);

b) Befestigen eines Sall-Linkers an dem DNA-Fragment (3) und anschließendes Verdauen mit Sall zur Gewinnung eines DNA-Fragments (4);

c) Verdauen des Plasmids pBR322 mit Sall zur Gewinnung eines DNA-Fragments (5) und

d) Verbinden der DNA-Fragmente (4) und (5) mittels T₄-Ligase zur Bildung des Plasmids pCR702.

9. Verfahren zur Herstellung des Plasmids pKSR101 in folgenden Stufen:

a) Verdauen des in E. coli gefundenen Plasmids pKS3 (hinterlegt bei der FRI unter der Hinterlegungsnummer FERM BP-503) mit BamHI und Sall zur Bildung eines DNA-Fragments (6);

b) vollständiges Verdauen des in E. coli gefundenen Plasmids pCR702 (hinterlegt bei der FRI unter der Hinterlegungsnummer FERM BP-501) mit BamHI und EcoRI zur Bildung eines DNA-Fragments (7);

c) Verdauen des in E. coli gefundenen Plasmids pCR702 (hinterlegt bei der FRI unter der Hinterlegungsnummer FERM BP-501) mit EcoRI und Sall zur Bildung eines DNA-Fragments (8) und

d) Verbinden der DNA-Fragmente (6), (7) und (8) mittels T₄-Ligase zur Bildung des Plasmids pKSR101.

10. In E. coli aufgefundenes Plasmid pKS3 (hinterlegt bei der FRI unter der Hinterlegungsnummer FERM BP-503).

11. In E. coli aufgefundenes Plasmid pCR702 (hinterlegt bei der FRI unter der Hinterlegungsnummer FERM BP-501).

12. In B. subtilis aufgefundenes Plasmid pKSR101 (hinterlegt bei der FRI unter der Hinterlegungsnummer FERM BP-504).

13. Bei der FRI unter der Hinterlegungsnummer FERM BP-503 hinterlegtes E. coli MC 1064 $r_k^- m_k^+ s_m^r$ - (pKS3).

14. Bei der FRI unter der Hinterlegungsnummer FERM BP-501 hinterlegtes E. coli MC1064 $r_k^- m_k^+ s_m^r$ - (pCR702).

15. Bei der FRI unter der Hinterlegungsnummer FERM BP-504 hinterlegtes B. subtilis RM125 $r^- m^-$, arg, leu (pKSR101).

# FIG. I

PROMOTER          29k PROTEIN GENE (ERYTHROMYCIN RESISTANT)

# F I G . 2

F I G. 3

FIG. 4